Europäisches Patentamt

European Patent Office

Office européen des brevets

(19)

(11) **EP 0 534 074 B1**

(12) **EUROPÄISCHE PATENTSCHRIFT**

(45) Veröffentlichungstag und Bekanntmachung des Hinweises auf die Patenterteilung:
06.03.1996 Patentblatt 1996/10

(51) Int. Cl.$^6$: **A61B 5/00**

(21) Anmeldenummer: 92112048.1

(22) Anmeldetag: 15.07.1992

(54) **Verfahren und Anordnung zur Bestimmung der Konzentration von Inhaltsstoffen in Körperflüssigkeiten**

Method and instrument for testing the concentration of body fluid constituents

Procédé et dispositif pour la mesure de la concentration des éléments constituants dans le fluide du corps

(84) Benannte Vertragsstaaten:
**AT CH DE FR GB IT LI NL SE**

(30) Priorität: **16.09.1991 DE 4130742**

(43) Veröffentlichungstag der Anmeldung:
**31.03.1993 Patentblatt 1993/13**

(73) Patentinhaber: **Institut für Diabetestechnologie Gemeinnützige Forschungs - und Entwicklungsgesellschaft mbH D-89081 Ulm (DE)**

(72) Erfinder:
• **Pfeiffer, Ernst F., Prof.Dr.Dres.h.c. W-7900 Ulm (DE)**
• **Meyerhoff, Carsten, Dr.med. W-7900 Ulm (DE)**
• **Zier, Horst W-7901 Lonsee-Luizhausen (DE)**
• **Keck, Fritz S., Priv.Doz.Dr.med. W-7909 Dornstadt (DE)**
• **Kerner, Wolfgang, Prof.Dr.med. W-7909 Dornstadt-Bollingen (DE)**

(74) Vertreter: **Wolf, Eckhard, Dr.-Ing. Patentanwälte Wolf & Lutz Hauptmannsreute 93 D-70193 Stuttgart (DE)**

(56) Entgegenhaltungen:
EP-A- 0 104 935        EP-A- 0 275 390
EP-A- 0 401 179        EP-A- 0 403 394

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren und eine Anordnung zur Bestimmung der Konzentration von Inhaltsstoffen, wie Glucose oder Milchsäure, in Gewebeflüssigkeit, wobei mittels einer im Körpergewebe implantierbaren, mit einem kontinuierlichen Perfusatstrom beaufschlagbaren Dialysesonde ein mit den Inhaltsstoffen angereicherter Dialysatstrom erzeugt und einer vorzugsweise extrakorporal angeordneten Meßanordnung zugeleitet wird.

Körpergewebe bestehen aus Zellen, die in einer Strömungsmittelumgebung leben, in der Nährstoffe und Zerfallsprodukte zwischen den Zellen und den Blutgefäßen transportiert werden. Die Konzentration der Inhaltsstoffe lassen somit Rückschlüsse auf den Stoffwechselzustand eines Patienten zu. Die Konzentrationsbestimmung kann beispielsweise durch Probenentnahme aus dem extrazellularen Raum nach dem Dialyseprinzip erfolgen. Hierzu wird eine Dialysesonde, die aus einer Dialysemembran und Leitungen für den Perfusatstrom besteht, in das Gewebe eingeführt. Das an der Membran vorbeiströmende Perfusat reichert sich mit Inhaltsstoffen aus der Gewebeflüssigkeit an und kann anschließend gesammelt und chemisch analysiert werden. In diesem Zusammenhang ist es auch bereits bekannt, das Dialysat unmittelbar einer Meßanordnung zuzuleiten (DE-OS 33 42 170).

Weiter ist es zur Bestimmung des Glucosegehalts in Gewebeflüssigkeit an sich bekannt (EP-A-275 390), eine in die Gewebeflüssigkeit eintauchende elektrochemische Enzymzelle unter Verwendung von Glucoseoxidase als Enzym zu verwenden und den Glucosegehalt aufgrund amperometrischer Messungen zu bestimmen. Umfangreiche Untersuchungen haben jedoch gezeigt, daß die unmittelbar in das Gewebe implantierte Enzymzelle in ihrer Meßempfindlichkeit mit der Zeit stetig abnimmt. Dies wird einmal auf reaktionshemmende Inhibitoren zurückgeführt, die in der Gewebeflüssigkeit enthalten sind und sich in der Enzymzelle ablagern. Zum anderen kann auch eine lokale Austrocknung des Gewebes im Bereich der implantierten Enzymzelle zu einer Verfälschung der Meßergebnisse führen.

Schließlich ist es an sich bekannt (EP-A 0 104 935), dem Perfusatstrom in Strömungsrichtung vor der Meßanordnung einen Pufferstrom zuzumischen. Allerdings werden bei dem dort beschriebenen Verfahren der Perfusatstrom und die zu untersuchende Substanz mittels einer Mischeinrichtung vermischt, so daß eine in-vivo-Anwendung nicht möglich ist.

Ausgehend hiervon liegt der Erfindung die Aufgabe zugrunde, das bekannte Verfahren der eingangs angegebenen Art dahingehend zu verbessern, daß kontinuierliche Konzentrationsmessungen über eine längere Zeit hinweg ohne Nacheichung möglich sind.

Zur Lösung dieser Aufgabe werden die in den Ansprüchen 1 bzw. 6 angegebenen Merkmalskombinationen vorgeschlagen. Weitere vorteilhafte Ausgestaltungen und Weiterbildungen der Erfindung ergeben sich aus den abhängigen Ansprüchen.

Die erfindungsgemäße Lösung geht von der bei vielen Meßreihen gewonnenen Erkenntnis aus, daß mit der Dialysemethode bei geeigneter Abstimmung zwischen Membrandurchlässigkeit, Membranoberfläche und Perfusatstrom sich nur eine geringe Störung im benachbarten Gewebebereich ergibt, so daß der unerwünschte Austrocknungseffekt vermieden wird. Andererseits hat es sich jedoch vor allem bei der Glucosebestimmung und der Milchsäurebestimmung gezeigt, daß bei unmittelbarem Eintauchen der elektrochemischen Enzymzelle in den Dialysatstrom sich nach wie vor ein zeitabhängiger Hemmfaktor bemerkbar macht, der innerhalb einer längerdauernden Meßserie eine Nacheichung der die Enzymzelle enthaltenden Meßschaltung erfordert. Um dies zu vermeiden, wird nun gemäß der Erfindung vorgeschlagen, daß dem Dialysatstrom in Strömungsrichtung vor der Meßanordnung ein zum Perfusatstrom proportionaler Pufferstrom zugemischt wird, daß mit dem so gebildeten Meßdialysat eine in der Meßanordnung angeordnete elektrochemische Enzymzelle kontinuierlich beaufschlagt wird, und daß der Perfusatstrom und der Pufferstrom aus einem gemeinsamen Flüssigkeitsreservoir angesaugt werden.

Dem Pufferstrom wird vorteilhafterweise ein phosphathaltiger pH-Stabilisator beigemischt. Vorteilhafterweise bestehen die Perfusionsflüssigkeit und/oder die Pufferflüssigkeit aus einer gegebenenfalls pH-stabilisierten physiologischen Kochsalzlösung oder aus Ringer-Lösung.

In der praktischen Anwendung hat sich ein Verhältnis zwischen Perfusatstrom und Pufferstrom von 1:0,5 bis 1:3 als optimal erwiesen. Der Perfusatstrom ist dabei auf die Dialysemembran-Oberfläche und deren Durchlässigkeit abzustimmen und beträgt zweckmäßig zwischen 1 und 5 µl/min bezogen auf eine Membranoberfläche von ca. 10 mm².

Zur Durchführung des erfindungsgemäßen Verfahrens wird vorteilhafterweise eine Anordnung verwendet, die eine in Körpergewebe implantierbare, die Gestalt eines doppellumigen Katheters aufweisende Dialysesonde enthält, die in der Nähe ihres distalen Endes eine geschlossene Dialysemembran trägt, und innerhalb der ein sich bis in den Membranbereich hinein erstreckender, mit einem kontinuierlichen Perfusatstrom beaufschlagbarer Zuflußkanal und ein entlang einer Dialysestrecke nach außen hin durch die Dialysemembran begrenzter, mit dem Zuflußkanal am distalen Ende kommunizierender, mit einer vorzugsweise extrakorporal angeordneten Meßanordnung verbundener Rückflußkanal angeordnet ist. Erfindungsgemäß weist hierbei die Meßanordnung eine in eine Durchflußkammer des Rückflußkanals eingreifende elektrochemische Enzymzelle auf, während in den Rückflußkanal in Strömungsrichtung vor der Enzymzelle ein mit einem zum Perfusatstrom proportionalen Pufferstrom beaufschlagbarer Bypass-Kanal mündet und wobei der Zuflußkanal und der Bypass-Kanal ansaugseitig mit einem gemeinsamen Flüssigkeitsreservoir verbunden sind.

Eine bevorzugte Ausgestaltung der Erfindung sieht vor, daß die Enzymzelle eine vorzugsweise aus Platin- oder Golddraht bestehende Meßelektrode, eine der Meßelektrode benachbarte, vorzugsweise aus Silber oder Edelstahl bestehende Bezugselektrode, sowie eine auf den Elektroden angeordnete, vorzugsweise mit einer semipermeablen Membran überzogene Enzymschicht aufweist. Je nach Anwendungsfall kann die Enzymschicht beispielsweise aus Glucoseoxidase (zur Bestimmung von β-D-Glucose) oder aus Lactatoxidase (zur Bestimmung von Lactat) bestehen. Zur Durchführung von Kombinationsmessungen kann auch eine Enzymzelle mit mindestens zwei mit unterschiedlichen Enzymen beschichteten oder unbeschichteten Meßelektroden und einer gemeinsamen Bezugselektrode eingesetzt werden. Die verschiedenen Meßelektroden greifen dabei zweckmäßig in getrennte Teilkanäle der Durchflußkammer ein.

Als Dialysemembran wird vorteilhafterweise eine mikroporige Polycarbonatfolie oder eine Polyurethanfolie mit einer Porenweite von ca. 0,01 μm verwendet.

Um eine exakte Proportionalität zwischen Perfusatstrom und Pufferstrom zu erzielen, sind der Zuflußkanal und der Bypass-Kanal vorteilhafterweise mit je einem elastisch nachgiebigen Ansaugschlauch einer gemeinsamen Rollendosierpumpe verbunden.

Um die durch die Dialysesonde hervorgerufenen Störungen innerhalb des Gewebes so klein wie möglich zu halten, wird gemäß einer vorteilhaften Ausgestaltung der Erfindung vorgeschlagen, sowohl den Zuflußkanal als auch den Rückflußkanal im Bereich der Dialysesonde aus elastisch nachgiebigem Kunststoffmaterial herzustellen, wobei notwendigerweise das Kunststoffmaterial etwas steifer als die Dialysemembran ausgebildet sein sollte.

Im folgenden wird die Erfindung anhand der Zeichnung näher erläutert. Es zeigen

Fig. 1    eine schematische Darstellung einer Meßanordnung zur Bestimmung der Konzentration von Inhaltsstoffen in Gewebeflüssigkeit nach der Dialysemethode;

Fig. 2    eine Dialysesonde in vergrößerter geschnittener Darstellung;

Fig. 3    einen Schnitt durch die Spitze eines Enzymsensors in einer Prinzipdarstellung.

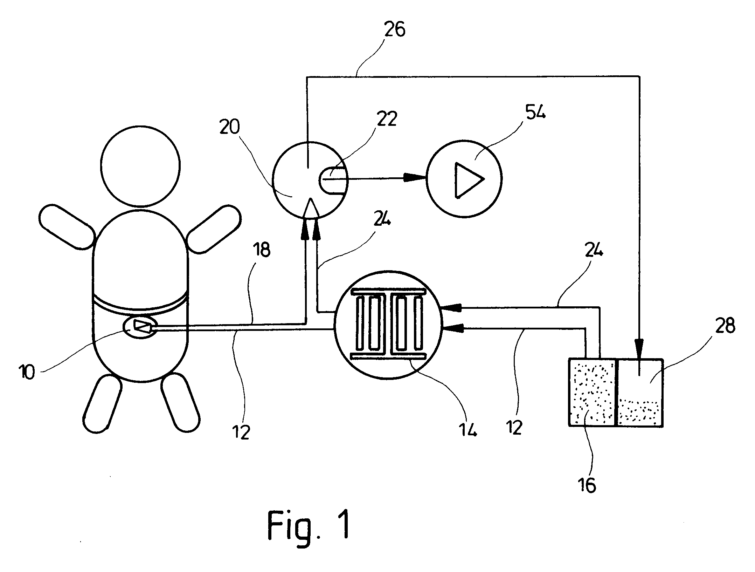

Die Meßanordnung gemäß Fig. 1 besteht im wesentlichen aus einer im subcutanen Gewebe implantierbaren Dialysesonde 10, die über einen Zuflußkanal 12 mit einer Perfusionsflüssigkeit beaufschlagbar ist, die mit Hilfe einer bei dem gezeigten Ausführungsbeispiel ausgebildeten Dosierpumpe 14 aus einem Flüssigkeitsreservoir 16 angesaugt wird. Die Perfusionsflüssigkeit besteht beispielsweise aus pH-stabilisierter physiologischer Kochsalzlösung oder Ringer-Lösung. Der Rückflußkanal 18 der Dialysesonde 10 führt zu einer Durchflußkammer 20, in die ein elektrochemischer Enzymsensor 22 eingreift. An die Durchflußkammer 20 ist eingangsseitig zusätzlich ein Bypass-Kanal 24 angeschlossen, der unmittelbar über das Flüssigkeitsreservoir 16 und die Rollendosierpumpe 14 mit einer Pufferlösung beaufschlagbar ist. Die auf die Zuflußleitung 12 und die Bypassleitung 24 gemeinsam einwirkende Rollendosierpumpe gewährleistet mit einfachen Mitteln die Einstellung eines vorgegebenen Verhältnisses zwischen Perfusatstrom und Pufferstrom nach Maßgabe der in der Pumpe verwendeten Schlauchquerschnitte im Zuflußkanal und Bypass-Kanal. Am Ausgang der Durchflußkammer wird die zuvor am Enzymsensor vorbeigeleitete Flüssigkeit gesammelt und über eine Sammelleitung 26 in einen Auffangbehälter 28 geleitet.

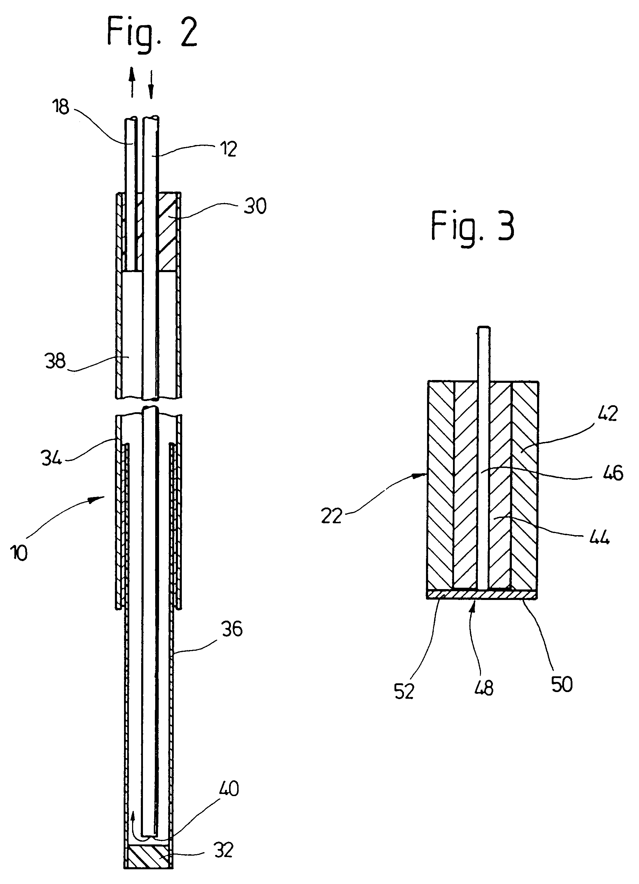

Wie aus Fig. 2 zu ersehen ist, besteht die Dialysesonde 10 im wesentlichen aus einer an ihrem proximalen und distalen Ende mittels eines Stopfens 30 und 32 verschlossenen Zylinderhülse 34, die im Bereich ihres distalen Endes eine Dialysemembran 36 aufweist und in die durch den proximalen Stopfen 30 hindurch der als dünnes Rohr ausgebildete Zuflußkanal 12 bis zum distalen Ende hindurchgreift. Der gleichfalls am proximalen Ende herausgeführte Rückflußkanal reicht über den Ringkanal 38 bis zum distalen Hülsenende und kommuniziert dort mit der Auslaßöffnung 40 des Zuflußkanals 12.

Der Membranteil 36 der Dialysesonde 10 ist bei deren Implantation vollständig in das Gewebe eingebettet, so daß das durch den Zuflußkanal 12 einströmende Perfusat durch die Membran hindurch mit Inhaltsstoffen aus dem interzellularen Gewebe befrachtet wird. Die Porosität der Dialysemembran 36 wird so bemessen, daß die zu bestimmenden Stoffwechselprodukte, wie Glucose oder Milchsäure nahezu widerstandsfrei in das Perfusat gelangen können, während größere Inhaltsstoffe zurückgehalten werden. Zur Glucose- und Milchsäurebestimmung wird die Porenweite bei 0,01 bis 0,03 μm gewählt.

Der in die Durchflußkammer 20 eingreifende, in Fig. 3 schematisch dargestellte Enzymsensor besteht im wesentlichen aus einem dünnen zylindrischen Rohr aus Silber oder Edelstahl, das eine isolierende Füllung 44, beispielsweise aus Epoxidharz sowie eine axial ausgerichtete Meßelektrode 46, vorzugsweise aus Platin oder Gold enthält. Der metallische Rohrmantel 42 bildet zugleich die Bezugselektrode des elektrochemischen Sensors.

Die aktive Meßstelle 48 des Sensors befindet sich an dessen Stirnfläche, an der die Meßelektrode 46 aus der Epoxidharz-Isolierung 44 herausragt und von einer semipermeablen Membran 50 und einem innerhalb der Membran eingeschlossenen Enzym 52 beschichtet ist. Die Bezugselektrode 42 und die Meßelekrode 46 sind mit einer in Fig. 1 symbolisch dargestellten Auswerteelektronik 54 verbunden, wie sie beispielsweise in der EP-A-275 390 beschrieben ist.

Bei der Meßdurchführung dient das in der Durchflußkammer 20 am Enzymsensor 22 vorbeigeführte Meßdialysat als Elektrolyt.

In einem Glucosesensor, der Glucoseoxidase als Enzym 52 enthält, wird im Meßdialysat vorhandene $\beta$-D-Glucose im Beisein von Sauerstoff in D-Lactone unter Freisetzung von $H_2O_2$ oxidiert:

$$\beta\text{-D-Glucose} + O_2 \xrightarrow{\text{GOD}} \text{D-Gluconolacton} + H_2O_2 \qquad (1)$$

Die Bildungsrate von $H_2O_2$ kann in der elektrochemischen Zelle als Diffusionsgrenzstrom aufgrund der folgenden Reaktion an der Platinanode gemessen werden:

$$H_2O_2 \xrightarrow{\text{Pt}} O_2 + 2H^+ + 2e^-. \qquad (2)$$

Der im Meßdialysat vorhandene Sauerstoff reicht für den Reaktionsablauf aus, zumal der bei der Glucose-Umwandlung verbrauchte Sauerstoff bei der Umwandlung von $H_2O_2$ an der Pt-Anode 46 zurückgewonnen und zumindest teilweise in das Meßdialysat zurückgeführt wird.

In dem Milchsäuresensor, der Lactatoxidase als Enzym 52 enthält, wird in dem Meßdialysat vorhandenes Lactat im Beisein von Sauerstoff in Pyruvat (Brenztraubensäure) unter Freisetzung von $H_2O_2$ oxidiert:

$$\text{Lactat} + O_2 \xrightarrow{\text{LOD}} \text{Pyruvat} + H_2O_2. \qquad (3)$$

Auch hier kann die Bildungsrate von $H_2O_2$ in der elektrochemischen Zelle als Diffusionsgrenzstrom im Sinne der vorstehenden Reaktionsgleichung (2) an der Platinanode gemessen werden.

Sowohl beim Glucosesensor als auch beim Lactatsensor wird der Diffusionsgrenzstrom mit einer positiven Polarisationsspannung gemessen.

Grundsätzlich ist es möglich, den in Fig. 1 gezeigten Sensor auch mit entgegengesetzter Polarisationsspannung, also mit dem Platindraht 46 als Kathode und dem Silberrohr 42 als Anode zu betreiben. Damit erhält man an der Meßelektrode eine Reduktion des im Meßdialysat enthaltenen Sauerstoffs zunächst zu $H_2O_2$ und anschließend zu $H_2O$ nach folgenden Reaktionsgleichungen:

$$O_2 + 2H^+ + 2e^- \xrightarrow{\text{Pt}} H_2O_2$$

$$H_2O_2 + 2H^+ + 2e^- \xrightarrow{\text{Pt}} 2H_2O.$$

Zur Messung des Sauerstoffpartialdrucks im Meßdialysat wird also der Differenzgrenzstrom bei konstanter Spannung zwischen Meßkathode und Bezugsanode als Maß für die in der Zeiteinheit die Kathode erreichenden $O_2$-Moleküle genutzt.

Zusammenfassend ist folgendes festzustellen: Die Erfindung bezieht sich auf ein Verfahren und eine Anordnung zur kontinuierlichen Bestimmung der Konzentration von Inhaltsstoffen, wie Glucose oder Milchsäure, in Körperflüssigkeiten. Die Probenentnahme aus dem Körpergewebe erfolgt nach dem Dialyseverfahren. Zu diesem Zweck ist eine im Körpergewebe implantierbare, die Gestalt eines doppellumigen Katheters aufweisende Dialysesonde 10 vorgesehen, die in der Nähe ihres distalen Endes eine geschlossene Dialyesmembran 26 trägt. Weiter ist ein sich innerhalb der Dialysesonde 10 bis in den Membranbereich hinein erstreckender, mit einem kontinuierlichen Perfusatstrom beaufschlagbarer Zuflußkanal 12 sowie ein entlang einer Dialysestrecke nach außen hin durch die Dialysemembran 36 begrenzter, mit dem Zuflußkanal am distalen Ende kommunizierender und mit einer Meßanordnung verbundener Rückflußkanal 18 vorgesehen. Um während einer möglichst langen Meßdauer driftfreie Konzentrationsmessungen durchführen zu können, weist die Meßanordnung eine in eine Durchflußkammer des Rückflußkanals eingreifende elektrochemische Enzymzelle 20 auf, während in den Rückflußkanal 18 in Strömungsrichtung vor der Enzymzelle 22 ein mit einem zum Perfusatstrom proportionalen Pufferstrom beaufschlagbarer Bypass-Kanal 24 mündet und wobei der Zuflußkanal und der Bypass-Kanal ansaugseitig mit einem gemeinsamen Flüssigkeitsreservoir verbunden sind.

## Patentansprüche

1. Verfahren zur Bestimmung der Konzentration von Inhaltsstoffen, wie Glucose oder Milchsäure, in Gewebeflüssigkeit, bei welchem mittels einer in Körpergewebe implantierbaren, mit einem kontinuierlichen Perfusatstrom beaufschlagbaren Dialysesonde (10) ein mit den Inhaltsstoffen angereicherter Dialysatstrom gebildet und einer vorzugsweise extrakorporal angeordneten Meßanordnung zugeleitet wird, **dadurch gekennzeichnet**, daß dem Dialysatstrom in Strömungsrichtung vor der Meßanordnung ein zum Perfusatstrom proportionaler Pufferstrom zugemischt wird, daß mit dem so gebildeten Meßdialysat eine in der Meßanordnung angeordnete elektrochemische Enzymzelle (22) kontinuierlich beaufschlagt wird, und daß der Perfusatstrom und der Pufferstrom aus einem gemeinsamen Flüssigkeitsreservoir (16) angesaugt werden.

**2.** Verfahren nach Anspruch 1, **dadurch gekennzeichnet,** daß das Verhältnis zwischen Perfusatstrom und Pufferstrom 1:0,5 bis 1:3 beträgt.

**3.** Verfahren nach einem der Ansprüche 1 oder 2, **dadurch gekennzeichnet**, daß der Perfusatstrom 1 bis 5 μl/min bezogen auf ca. 10 mm² Dialysemembran-Oberfläche beträgt.

**4.** Verfahren nach einem der Ansprüche 1 bis 3, **dadurch gekennzeichnet**, daß dem Pufferstrom ein phosphathaltiger pH-Stabilisator beigemischt wird.

**5.** Verfahren nach einem der Ansprüche 1 bis 4, **dadurch gekennzeichnet**, daß das Perfusat und/oder die Pufferflüssigkeit aus gegebenenfalls pH-stabilisierter physiologischer Kochsalzlösung oder Ringer-Lösung bestehen.

**6.** Anordnung zur kontinuierlichen Bestimmung der Konzentration von Inhaltsstoffen, wie Glucose oder Milchsäure, in Körperflüssigkeiten, mit einer in Körpergewebe implantierbaren, die Gestalt eines doppellumigen Katheters aufweisenden Dialysesonde (10), die in der Nähe ihres distalen Endes eines geschlossene Dialysemembran (36) trägt, mit einem sich innerhalb der Dialysesonde bis in den Membranbereich erstreckenden, mit einem kontinuierlichen Perfusatstrom beaufschlagbaren Zuflußkanal (12) und einem entlang einer Dialysestrecke nach außen hin durch die Dialysemembran (36) begrenzten, mit dem Zuflußkanal (12) am distalen Ende kommunizierenden, mit einer vorzugsweise extrakorporal angeordneten Meßanordnung verbundenen Rückflußkanal (18), **dadurch gekennzeichnet**, daß die Meßanordnung eine in eine Durchflußkammer (20) des Rückflußkanals (18) eingreifende elektrochemische Enzymzelle (22) aufweist, daß in den Rückflußkanal (18) in Strömungsrichtung vor der Enzymzelle (22) ein mit einem zum Perfusatstrom proportionalen Pufferstrom beaufschlagbarer Bypass-Kanal (24) mündet, und daß der Zuflußkanal (12) und der Bypass-Kanal (24) ansaugseitig mit einem gemeinsamen Flüssigkeitsreservoir (16) verbunden sind.

**7.** Anordnung nach Anspruch 6, **dadurch gekennzeichnet**, daß die Enzymzelle (22) eine vorzugsweise aus Platin- oder Golddraht bestehende Meßelektrode (46), eine der Meßelektrode (46) benachbarte, vorzugsweise aus Silber oder Edelstahl bestehende Bezugselektrode (42), sowie eine auf den Elektroden angeordnete, vorzugsweise mit einer semipermeablen Membran (50) überzogene Enzymschicht (32) aufweist.

**8.** Anordnung nach Anspruch 7, **dadurch gekennzeichnet**, daß das Enzym (52) aus immobilisierter Glucoseoxidase (GOD) besteht.

**9.** Anordnung nach Anspruch 7, **dadurch gekennzeichnet**, daß das Enzym (52) aus immobilisierter Lactatoxidase (LOD) besteht.

**10.** Anordnung nach einem der Ansprüche 6 bis 9, **dadurch gekennzeichnet**, daß die Enzymzelle (22) mindestens zwei mit unterschiedlichen Enzymen beschichtete oder unbeschichtete Meßelektroden und eine gemeinsame Bezugselektrode aufweist.

**11.** Anordnung nach Anspruch 10, **dadurch gekennzeichnet**, daß die verschiedenen Meßelektroden der Enzymzelle in getrennte Teilkanäle der Durchflußkammer eingreifen.

**12.** Anordnung nach einem der Ansprüche 6 bis 11, **dadurch gekennzeichnet**, daß die Dialysemembran (36) aus mikroporiger Polycarbonat-Folie mit einer Porenweite von ca. 0,01 μm besteht.

**13.** Anordnung nach einem der Ansprüche 6 bis 12, **dadurch gekennzeichnet**, daß der Zuflußkanal (12) und der Bypass-Kanal (24) mit je einem elastisch nachgiebigen Ansaugschlauch einer gemeinsamen Rollendosierpumpe (14) verbunden sind.

**14.** Anordnung nach einem der Ansprüche 6 bis 13, **dadurch gekennzeichnet**, daß der Zuflußkanal (12) innerhalb der Dialysesonde (10) aus einem elastischen Kunststoffschlauch bestehen.

## Claims

**1.** A process for determining the concentration of constituents, such as glucose or lactic acid, in tissue fluid, in which a dialysate stream enriched with the constituents is formed by means of a dialysis probe (10), which can be supplied with a continuous perfusate stream and can be implanted in body tissue, and is passed into a preferably extracorporeally located measuring arrangement, characterised in that the dialysate stream is mixed with a buffer stream proportional to the perfusate stream in the direction of flow before the measuring arrangement, that an electrochemical enzyme cell (22) located in the measuring arrangement is continuously supplied with the measuring dialysate formed in this way, and that the perfusate stream and the buffer stream are drawn by suction from a common liquid reservoir (16).

2. A process according to Claim 1, characterised in that the ratio between perfusate stream and buffer stream is 1:0.5 to 1:3.

3. A process according to one of Claims 1 or 2, characterised in that the perfusate stream is 1 to 5 µl/min with respect to ca. 10 mm$^2$ of dialysis membrane surface area.

4. A process according to one of Claims 1 to 3, characterised in that the buffer stream is mixed with a phosphate-containing pH stabiliser.

5. A process according to one of Claims 1 to 4, characterised in that the perfusate and/or the buffer liquid comprises optionally pH stabilised physiological saline solution or Ringer's solution.

6. An arrangement for the continuous determination of the concentration of constituents, such as glucose or lactic acid, in body fluids, with a dialysis probe (10) in the form of a double-lumen catheter, which can be implanted in body tissue, which has a sealed dialysis membrane (36) in the vicinity of its distal end, with a supply channel (12) which can be supplied with a continuous perfusate stream extending inside the dialysis probe down to the membrane region and a return channel (18) bounded on the outside along the dialysis section by the dialysis membrane (36), communicating with supply channel (12) at its distal end, connected to a preferably extracorporeally located measuring arrangement, characterised in that the measuring arrangement has an electrochemical enzyme cell (22) inserted into a flow-chamber (20) in the return channel (18), that a by-pass channel (24) which can be supplied with a buffer stream which is proportional to the perfusate stream enters return channel (18) in the direction of flow before the enzyme cell (22), and that the supply channel (12) and the by-pass channel (24) are connected on the suction side to a common liquid reservoir (16)

7. An arrangement according to Claim 6, characterised in that the enzyme cell (22) has a measuring electrode (46) preferably consisting of platinum or gold wire, a reference electrode (42), preferably consisting of silver or stainless steel, alongside the measuring electrode (46) and an enzyme layer (32) located on the electrodes, preferably coated with a semipermeable membrane (50).

8. An arrangement according to Claim 7, characterised in that the enzyme (52) consists of immobilised glucoseoxidase (GOD).

9. An arrangement according to Claim 7, characterised in that the enzyme (52) consists of immobilised lactatoxidase (LOD).

10. An arrangement according to one of Claims 6 to 9, characterised in that the enzyme cell (22) has at least two measuring electrodes, coated with different enzymes or uncoated, and a common reference electrode.

11. An arrangement according to Claim 10, characterised in that the different measuring electrodes in the enzyme cell are inserted into separate sub-channels in the flow-chamber.

12. An arrangement according to one of Claims 6 to 11, characterised in that the dialysis membrane (36) consists of microporous polycarbonate film with a pore-size of ca. 0.01 µm.

13. An arrangement according to one of Claims 6 to 12, characterised in that the supply channel (12) and the by-pass channel (24) are each connected to a common peristaltic metering pump (14) with elastically resilient suction tubing.

14. An arrangement according to one of Claims 6 to 13, characterised in that the supply channel (12) inside the dialysis probe (10) consists of elastic synthetic tubing.

**Revendications**

1. Procédé pour la mesure de la concentration de constituants, tels que glucose ou acide lactique, dans du liquide tissulaire, selon lequel un courant de dialysat enrichi en constituants est formé au moyen d'une sonde de dialyse (10), qui peut être implantée dans le tissu organique et être alimentée par un courant de perfusat continu, et est dirigé dans un montage de mesure, extra-corporel de préférence, caractérisé en ce qu'un courant tampon, proportionnel au courant de perfusat, est mélangé au courant de dialyse, en amont du montage de mesure dans le sens d'écoulement, en ce qu'une cellule enzymatique électrochimique (22), disposée dans le montage de mesure, est alimentée en continu par le dialysat ainsi formé, et en ce que le courant de perfusat et le courant tampon sont aspirés d'un réservoir de liquide commun (16).

2. Procédé suivant la revendication 1, caractérisé en ce que le rapport entre le courant de perfusat et le courant tampon est de 1 : 0,5 à 1 : 3.

3. Procédé suivant l'une des revendications 1 ou 2, caractérisé en ce que le courant de perfusat est de 1 à 5 $\mu$l/min, rapporté à une surface de la membrane de dialyse de l'ordre de 10 mm$^2$.

4. Procédé suivant l'une des revendications 1 à 3, caractérisé en ce qu'un stabilisant de pH phosphaté est mélangé au courant tampon.

5. Procédé suivant l'une des revendications 1 à 4, caractérisé en ce que le perfusat et/ou le liquide tampon se composent d'eau salée physiologique, éventuellement stabilisée en pH, et de liquide de Ringer.

6. Dispositif pour la mesure en continu de la concentration de constituants, tels que glucose ou acide lactique, dans des liquides organiques, avec une sonde de dialyse (10) qui peut être implantée dans le tissu organique, présente la forme d'un cathéter à double lumen, et supporte, au voisinage de son extrémité distale, une membrane de dialyse fermée (36), avec un canal d'alimentation (12), s'étendant à l'intérieur de la sonde de dialyse jusque dans la zone de la membrane et pouvant être alimenté par un courant continu de perfusat, et avec un canal de retour (18), délimité en direction de l'extérieur par la membrane de dialyse (36) le long d'un parcours de dialyse, communiquant avec le canal d'alimentation (12) sur l'extrémité distale, et relié à un montage de mesure extra-corporel de préférence, caractérisé en ce que le montage de mesure présente une cellule enzymatique électrochimique (22), s'engageant dans un compartiment de passage (20) du canal de retour (18), en ce qu'un canal de bipasse (24), pouvant être alimenté par un courant tampon proportionnel au courant de perfusat, débouche dans le canal de retour (18), en amont de la cellule enzymatique (22) dans le sens d'écoulement, et en ce que le canal d'alimentation (12) et le canal de bipasse (24) sont reliés, côté aspiration, à un réservoir de liquide commun (16).

7. Dispositif suivant la revendication 6, caractérisé en ce que la cellule enzymatique (22) présente une électrode de mesure (46), en fil de platine ou d'or de préférence, une électrode de référence (42), en argent eu en acier spécial de préférence, limitrophe à l'électrode de mesure (46), ainsi qu'une couche d'enzyme (52), disposée sur les électrodes et revêtue de préférence d'une membrane (50) semi-perméable.

8. Dispositif suivant la revendication 7, caractérisé en ce que l'enzyme (52) se compose d'oxydase de glucose immobilisée (GOD).

9. Dispositif suivant la revendication 7, caractérisé en ce que l'enzyme (52) se compose d'oxydase de lactate immobilisée (LOD).

10. Dispositif suivant l'une des revendications 6 à 9, caractérisé en ce que la cellule enzymatique (22) présente au moins deux électrode de mesure, revêtues ou non revêtues de différentes enzymes, et une électrode de référence commune.

11. Dispositif suivant la revendication 10, caractérisé en ce que les différentes électrodes de mesure de la cellule enzymatique s'engagent dans des canaux partiels séparés du compartiment de passage.

12. Dispositif suivant l'une des revendications 6 à 11, caractérisé en ce que la membrane de dialyse (36) se compose d'un film de polycarbonate microporeux, d'une ouverture de pore de l'ordre de 0,01 $\mu$m.

13. Dispositif suivant l'une des revendications 6 à 12, caractérisé en ce que le canal d'alimentation (12) et le canal de bipasse (24) sont reliés à un flexible d'aspiration respectif, élastique et souple, d'une pompe doseuse à rouleaux (14) commune.

14. Dispositif suivant l'une des revendications 6 à 13, caractérisé en ce que le canal d'alimentation (12) se compose, à l'intérieur de la sonde de dialyse (10), d'un flexible de matière plastique élastique.

Fig. 1

# Fig. 2

# Fig. 3